# EUROPEAN PATENT APPLICATION

(11) **EP 4 186 563 A1**
(43) Date of publication of application: **31.05.2023**
(21) Application number: 21846163.0
(22) Date of filing: 21.07.2021
(51) Int. Cl.: A61P 31/02, A61P 31/14, C01B 11/08, A61P 17/00, A61L 2/18, A61K 33/20, A01N 59/08

(54) **CORONA VIRUS KILLING AGENT**

(30) Priority: 22.07.2020 JP 2020125824
(71) Applicant: Sankei Co., Ltd., Chuo-ku Osaka-shi Osaka 540-0001 (JP)
(72) Inventor: GODA, Hisataka, Osaka-shi, Osaka 541-0048 (JP)
(74) Representative: Müller-Boré & Partner Patentanwälte PartG mbB
(86) International application number: PCT/JP2021/027361
(87) International publication number: WO 2022/019334

(57) **Abstract**

The present disclosure provides a corona virus killing agent. In particular, the present disclosure provides a corona virus killing agent containing chlorous acid water. In particular, the present disclosure provides a method for killing corona virus, using chlorous acid water. The free chlorine concentration of the chlorous acid water (as Cl = 35.45) may be at least 5 ppm or greater in the absence of organic matter. The content of chlorous acid in the chlorous acid water (as HClO₂ = 68.46) may be at least 200 ppm or greater in the presence of organic matter such as when disinfecting hands/fingers. The corona virus may be SARS corona virus (SARS-CoV), MERS corona virus (MERS-CoV), or the 2019 novel corona virus (SARS-CoV-2).

## Description

### [Technical Field]

The present disclosure relates to a coronavirus-killing agent. The present disclosure also relates to a method of killing or inactivating a coronavirus. The present disclosure also relates to a chlorous acid aqueous solution for killing or inactivating a coronavirus.

### [Background Art]

Coronavirus infections have become an issue in recent years. Humans are routinely infected by four types of coronaviruses (Human Coronavirus: HCoV), i.e., HCoV-229E, HCoV-OC43, HCoV-NL63, and HCoV-HKU1. While many of the infected patients have mild symptoms, some may experience high fever. Severe acute respiratory syndrome coronavirus (SARS-CoV) originated in Guangdong Province in China in 2002, and spread to over 30 countries and regions from November 2002 to July 2003 (case fatality rate: 9.60). Although the Middle East respiratory syndrome coronavirus (MERS-CoV) is a virus that induces cold-like symptoms in Arabian camels, it is understood that the virus induces severe pneumonia when a human is infected beyond the barrier of species (case fatality rate: 34.4%). 2019 novel coronaviruses are induced by SARS-CoV-2 viruses (known as COVID-19 viruses). The world is facing an unprecedented threat from a worldwide pandemic.

The primary active ingredient of a chlorous acid aqueous solution is chlorous acid (HClO₂) (Patent Literature 1).

### [Citation List]

### [Patent Literature]

[PTL 1] International Publication No. WO 2008/026607

### [Summary of Invention]

### [Solution to Problem]

The inventors discovered that chlorous acid aqueous solutions are effective in killing coronaviruses as a result of diligent studies to complete the present disclosure. The inventors also discovered that chlorous acid aqueous solutions are effective in killing 2019 novel coronaviruses to complete the present disclosure.

According to the Ministry of Health, Labour and Welfare with regard to chlorine-based disinfectants, sterilizers, or microbicides, sodium hypochlorite is unsuitable for application on hands and fingers, while the effect of hypochlorous acid water on hands and finger is unknown, so that use thereof is considered unsuitable.

For example, the present invention provides the following items.

### (Item 1)

An agent for use in killing coronavirus comprising a chlorous acid aqueous water.

### (Item 2)

The agent for use in killing of item 1, wherein a free chlorine concentration (as Cl = 35.45) of the chlorous acid aqueous solution is at least 1 mg/L in the absence of an organic matter.

### (Item 3)

The agent for use in killing of item 1, wherein chlorous acid content (as HClO₂ = 68.46) of the chlorous acid aqueous solution is at least 100 ppm in the absence of an organic matter.

### (Item 4)

The agent for use in killing of item 1, wherein a free chlorine concentration (as Cl = 35.45) of the chlorous acid aqueous solution is at least 5 mg/L in the presence of an organic matter including disinfection of a hand, finger, etc.

### (Item 5)

The agent for use in killing of item 1, wherein chlorous acid content (as HClO₂ = 68.46) of the chlorous acid aqueous solution is at least 200 ppm in the presence of an organic matter including disinfection of a hand, finger, etc.

### (Item 6)

The agent for use in killing of item 1, wherein a free chlorine concentration (as Cl = 35.45) of the chlorous acid aqueous solution is at least 10 mg/L in the presence of an organic matter including a contaminant, etc. equivalent to 0.5% BSA or greater.

### (Item 7)

The agent for use in killing of item 1, wherein chlorous acid content (as HClO₂ = 68.46) of the chlorous acid aqueous solution is at least 400 ppm in the presence of an organic matter including a contaminant, etc. equivalent to 0.5% BSA or greater.

### (Item 8)

The agent for use in killing of item 7, wherein a specification value of the chlorous acid aqueous solution is 4 to 6%.

### (Item 9)

The agent for use in killing of any one of items 1 to 8, wherein the coronavirus is a Letovirinae or Orthocoronavirinae virus.

### (Item 10)

The agent for use in killing of any one of items 1 to 9, wherein the coronavirus is a virus of the genus alphacoronavirus, betacoronavirus, gamma corona virus, or deltacoronavirus.

### (Item 11)

The agent for use in killing of any one of items 1 to 10, wherein the coronavirus is a virus of the genus betacoronavirus.

### (Item 12)

The agent for use in killing of any one of items 1 to 11, wherein the coronavirus is a virus of the subgenus Colacovirus, Decacovirus, Duvinacovirus, Luchacovirus, Minacovirus, Minunacovirus, Myotacovirus, Nyctacovirus, Pedacovirus, Rhinacovirus, Setracovirus, Soracovirus, Sunacovirus, Tegacovirus, Embecovirus, Hibecovirus, Merbecovirus, Nobecovirus, Sarbecovirus, Andecovirus, Buldecovirus, Herdecovirus, Brangacovirus, Cegacovirus, or Igacovirus.

### (Item 13)

The agent for use in killing of any one of items 1 to 12, wherein the coronavirus is a coronavirus that infects a human.

### (Item 14)

The agent for use in killing of any one of items 1 to 13, wherein the coronavirus is HCoV-HKU1, HCoV-OC43, SARS coronavirus (SARS-CoV), MERS coronavirus (MERS-CoV), or 2019 novel coronavirus (SARS-CoV-2).

### (Item 15)

The agent for use in killing of any one of items 1 to 14, wherein the coronavirus is SARS coronavirus (SARS-CoV), MERS coronavirus (MERS-CoV), or 2019 novel coronavirus (SARS-CoV-2) .

### (Item 16)

The agent for use in killing of any one of items 1 to 15, wherein the agent for use in killing is an agent for use in killing for hands and fingers, and a free chlorine concentration (as Cl = 35.45) of the chlorous acid aqueous solution is at least 5 mg/L.

### (Item 17)

A method of killing a coronavirus by using a chlorous acid aqueous solution.

### (Item 18)

The method of item 17, wherein the coronavirus is contacted with the chlorous acid aqueous solution in the absence of an organic matter.

### (Item 19)

The method of item 17 or 18, wherein a free chlorine concentration (as Cl = 35.45) of the chlorous acid aqueous solution is at least 1 mg/L.

### (Item 20)

The method of any one of items 17 to 19, wherein the coronavirus is contacted with the chlorous acid aqueous solution in the presence of an organic matter.

### (Item 21)

The method of any one of items 17 to 20, wherein a free chlorine concentration (as Cl = 35.45) of the chlorous acid aqueous solution is at least 10 mg/L.

### (Item 22)

A chlorous acid aqueous solution for use in killing a coronavirus.

The present disclosure is intended so that one or more of the features described above can be provided not only as the explicitly disclosed combinations, but also as other combinations. Additional embodiments and advantages of the present disclosure are recognized by those skilled in the art by reading and understanding the following detailed description as needed.

### [Advantageous Effects of Invention]

A coronavirus can be killed.

### [Brief Description of Drawings]

[Figure 1] Figure **1** shows the results of a test on an antiviral effect of a chlorous acid aqueous solution against SARS-CoV-2 in the absence of an organic matter. Under each condition, the left side is No-virus, and the right side is SARS-CoV-2.
[Figure 2] Figure **2** shows the results of a test on an antiviral effect of a chlorous acid aqueous solution against SARS-CoV-2 in the presence of an organic matter. Under each condition, the left side is No-virus, and the right side is SARS-CoV-2.

### [Description of Embodiments]

The present disclosure is described in more detail hereinafter. Throughout the entire specification, a singular expression should be understood as encompassing the concept thereof in the plural form, unless specifically noted otherwise. Thus, singular articles (e.g., "a", "an", "the", and the like in case of English) should also be understood as encompassing the concept thereof in the plural form, unless specifically noted otherwise. The terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

The abbreviations used herein have the conventional meaning within the scope of the art unless specifically noted otherwise.

The term "about" for a value or parameter herein includes variations about the value or parameter itself. Unless specifically noted otherwise, "about X" for example includes "X" itself as well as values with an acceptable error of ± 10% therefrom.

As used herein, "chlorous acid aqueous solution" refers to an aqueous solution comprising chlorous acid (HClO₂) used as a sterilizing agent, which can stably maintain chlorous acid (HClO₂) over a long period of time. The presence of a chlorous acid aqueous solution can be confirmed if an absorbent section comprising an acidic chlorite ion (H⁺+ClO₂⁻) representing a peak near 260 nm and an absorbent section comprising chlorine dioxide (ClO₂) representing a peak near 350 nm can be simultaneously observed between wavelengths of 240 to 420 nm in the UV spectrum, i.e., if a double peak is exhibited, when a chlorous acid aqueous solution sample is measured with a spectrophotometer.

As used herein, "specification value of chlorous acid aqueous solution" refers to a value of the concentration of chlorous acid in a chlorous acid aqueous solution calculated by a predetermined method. The same value is calculated by using the method described in the Examples. Generally, a chlorous acid aqueous solution with a specification value of 4 to 6 can be used as an active pharmaceutical ingredient, and chlorous acid aqueous solution formulations using the same can include 4 to 6% raw material chlorous acid aqueous solution products.

As the definition of chlorous acid content (HClO₂ = 68.46), "chlorous acid content (HClO₂ = 68.46)" as used herein is used in the same meaning as the common usage in the art, and refers to, in the context of a chlorine-based product (e.g., chlorous acid aqueous solution), a value obtained by an iodometric method. This value is a value converted in terms of chlorous acid (HClO₂ = 68.46), and refers to such a concentration (e.g., ppm, etc.) indicated as content.

A chlorous acid aqueous solution can be prepared by the methods disclosed in International Publication No. WO 2008/026607, WO 2014/188310, WO 2014/188311, WO 2014/188312, WO 2015/093062, or WO 2017/170904.

"Chlorous acid aqueous solution" is a sterilizer that was designated as a food additive on February 1, 2013 and has chlorous acid (HClO₂) as the primary active ingredient. The primary active ingredient chlorous acid (HClO₂) of such a "chlorous acid aqueous solution" is a semi-stable chemical substance, which is approved by the USDA and FDA as a food additive: processing aid as an especially safe substance. The chlorous acid aqueous solution used in the present disclosure that does not fall under a food additive can also be used and can be provided as a pharmaceutical product, quasi-drug, or various other products.

Moreover, "chlorous acid aqueous solution" can exert a potent sterilizing effect even in the presence of organic matter. Chlorous acid aqueous solution was highly praised as "only chlorous acid aqueous solution was capable of inactivation to the detection limit or below under all load conditions" in "Heisei 27 Nendo Norouirusu no Fukatsuka Joken ni Kansuru Chosa Hokokusho [2015 Investigative Report on Norovirus Inactivation Conditions]" at the National Institute of Health Sciences (NIHS). With the revisions of the Ordinance for Enforcement of the Food Sanitation Act, listing of chlorous acid aqueous solutions is ongoing in "Tairyo Chori Shisetsu Chori Manyuaru [Manual for Food Preparation at Large-scale Food Preparation Facilities]" and "Tsukemono no Eisei Kihan [Code of Hygienic Practice for Pickles]", etc., in order of incidents of large scale food poisoning.

In addition, chlorous acid aqueous solutions were approved as a pharmaceutical product as a class II sterilizing disinfectant in 2019, with a scheduled sale in 2020. Chlorous acid aqueous solution is added to guidelines such as "Hoikusho ni okeru Kansen Taisaku Gaidorain [Infection Control Measure Guideline at Nursery Center]" under the administration of the Ministry of Health, Labour and Welfare including "Norouirusu ni Kansuru Q&A [Q&A regarding noroviruses]", relevant manuals such as "Koreisha Kaigo Shien Shisetsu ni okeru Kansen Taisaku Manyuaru [Manual for Controlling Infection at Elderly Care Support Facility]", various sanitation codes such as "Bento/Sozai no Eisei Kihan [Code of Sanitation for Bento/Dishes]", etc., which are also being revised as needed. A chlorous acid aqueous solution is a substance that is supplied to wide-ranging markets in food sanitation and environmental sanitation markets in Japan.

"Chlorous acid aqueous solution" with chlorous acid as the primary active ingredient was found to possess a better coronavirus-killing (disinfecting/microbicidal) action than other chlorine-based agents such as "hypochlorous acid water" and "sodium hypochlorite". In this manner, a chlorous acid aqueous solution has a characteristic of possessing a potent sterilizing power, but gradual reactivity such that it can be used on hands and fingers, with no instantaneous sterilizing effect (immediate effect), but gradual reactivity while having a precise as well as sustained stable sterilizing power. "Chlorous acid aqueous solution" can exert a sterilizing effect that is precise and accurate, albeit slowly, under a contaminated environment with a large quantity of organic matter, which had been considered the most challenging for chlorine oxide-based agents (sterilizing power against microorganisms that are latent in contamination). For example, the effect of "chlorous acid aqueous solution" in the presence of an organic matter is listed in "Heisei 27 Nendo Norouirusu no Fukatsuka Joken ni Kansuru Chosa Hokokusho [2015 Investigative Report on Norovirus Inactivation Conditions] (National Institute of Health Sciences, Division of Biomedical Food Research)" on the website of the Ministry of Health, Labour and Welfare.

"Chlorous acid aqueous solution" does not need to be prepared upon use or require a dedicated generator, etc. A chlorous acid aqueous solution is safe and can be used anywhere by anyone whenever use thereof is desirable.

As used herein, "chlorous acid aqueous solution formulation" refers to a formulation that is prepared by using a chlorous acid aqueous solution as an active pharmaceutical ingredient. The additional component, pH, chlorous acid content, free chlorine concentration, etc. can be adjusted depending on the application.

As used herein, "active pharmaceutical ingredient chlorous acid aqueous solution" refers to a chlorous acid aqueous solution used as an active pharmaceutical ingredient. A chlorous acid aqueous solution can be prepared by a method described herein.

Since a "chlorous acid aqueous solution formulation" used in the present disclosure has low stimulation even when directly contacted with an animal, the formulation can be a formulation that does not cause any damage. A chlorous acid aqueous solution formulation used in the present disclosure can be a formulation that does not result in a skin corrosive reaction, erythema, or edema when applied to the skin, maintains normal cornea, iris, and conjunctiva when applied to the eye, and does not exhibit skin sensitivity.

As used herein, chlorous acid aqueous solution content is a value measured by an iodometric method, and "free chlorine", "free chlorine concentration", or "free residual chlorine concentration" is a value that is measured according to Appendix 3 in "Testing method for free residual chlorine and bound chlorine specified by the Minister of Health, Labour and Welfare based on the provision of Article 17(2) of the regulation of the Water Supply Act" (hereinafter, colorimetry (DPD indicator)), and is a value obtained by oxidation of a DPD indicator.

As used herein, "contaminant equivalent to 0.5% BSA or greater" indicates a contaminant equivalent to an organic matter used in a load test described in "Heisei 27 Nendo Norouirusu no Fukatsuka Joken ni Kansuru Chosa Hokokusho [2015 Investigative Report on Norovirus Inactivation Conditions], National Institute of Health Sciences, Division of Biomedical Food Research". The organic matter load test is described as mixing a solution diluted with a MEM medium to comprise bovine serum albumin BSA (Sigma, A9576-50ML), meat extract (Nacalai Tesque, 15837-55), and polypeptone (Nihon Pharmaceutical, HiPolypepton N, 397-02121) at 10% with a viral solution at 1:1 and using the mixture as a 5% organic matter supplemented viral solution. BSA is an abbreviation for bovine serum albumin.

As used herein, "in the presence of an organic matter including disinfection of a hand, finger, etc." indicates a condition under which an organic matter that can be normally present in a hand, finger, etc. is present. This corresponds to a case where "contaminant equivalent to 0.5% BSA or greater" is present.

As used herein, "in the absence of an organic matter" indicates complete absence of an organic matter, as well as substantial absence (including a value at or below a threshold value). This refers to any "state which is not in the presence of an organic matter".

### (Preferred Embodiments)

In one aspect of the present disclosure, a coronavirus-killing agent comprising a chlorous acid aqueous solution is provided. According to the website of the Ministry of Health, Labour and Welfare for chlorine-based disinfectants, sterilizers, or microbicides, sodium hypochlorite is unsuitable for application on hands and fingers. The effect of hypochlorous acid water on hands and finger is unknown, and use thereof is considered unsuitable. Thus, the application of the chlorous acid aqueous solution of the present disclosure as a coronavirus-killing agent is unexpected in view of being a chlorine-based sterilizing agent, and it was unexpected that coronaviruses including SARS-CoV-2 can be killed in view of the effect of conventionally known chlorous acid aqueous solutions. This is significantly different from, and advantageous over other chlorine-based disinfectants in that the coronavirus-killing agent of the present disclosure is demonstrated to be usable by directly spraying or immersing hands and fingers when used in the same manner as alcohol for use in daily disinfection.

The present disclosure also provides a killing agent wherein a free chlorine concentration (as Cl = 35.45) of the chlorous acid aqueous solution is at least 1 mg/L in the absence of an organic matter. The present disclosure also provides a killing agent wherein chlorous acid content (as HClO₂ = 68.46) of the chlorous acid aqueous solution is at least 100 ppm in the absence of an organic matter. The free chlorine concentration (as Cl = 35.45) of the chlorous acid aqueous solution in the absence of an organic matter can be at least 1 mg/L, at least 5 mg/L, at least 10 mg/L, at least 20 mg/L, at least 30 mg/L, at least 40 mg/L, at least 50 mg/L, at least 60 mg/L, at least 70 mg/L, at least 80 mg/L, at least 90 mg/L, at least 100 mg/L, at least 500 mg/L, at least 1000 mg/L, at least 2000 mg/L, at least 3000 mg/L, at least 4000 mg/L, at least 5000 mg/L, at least 6000 mg/L, at least 7000 mg/L, at least 8000 mg/L, at least 9000 mg/L, or at least 10,000 mg/L.

The present disclosure also provides a killing agent wherein a free chlorine concentration (as Cl = 35.45) of the chlorous acid aqueous solution is at least 5 mg/L to 200 mg/L in the presence of an organic matter including disinfection of a hand, finger, etc. The present disclosure also provides a killing agent wherein chlorous acid content (as HClO₂ = 68.46) of the chlorous acid aqueous solution is at least 200 ppm in the presence of an organic matter including disinfection of a hand, finger, etc. The free chlorine concentration (as Cl = 35.45) of the chlorous acid aqueous solution in the presence of an organic matter including disinfection of a hand, finger, etc. can be at least 5 mg/L, at least 10 mg/L, at least 20 mg/L, at least 30 mg/L, at least 40 mg/L, at least 50 mg/L, at least 60 mg/L, at least 70 mg/L, at least 80 mg/L, at least 90 mg/L, at least 100 mg/L, at least 110 mg/L, at least 120 mg/L, at least 130 mg/L, at least 140 mg/L, at least 150 mg/L, at least 160 mg/L, at least 170 mg/L, at least 180 mg/L, at least 190 mg/L, or at least 200 mg/L.

The present disclosure also provides a killing agent wherein a free chlorine concentration (as Cl = 35.45) of the chlorous acid aqueous solution is at least 10 mg/L in the presence of an organic matter including a contaminant, etc. equivalent to 0.5% BSA or greater. The present disclosure also provides a killing agent wherein chlorous acid content (as HClO₂ = 68.46) of the chlorous acid aqueous solution is at least 400 ppm in the presence of an organic matter including a contaminant, etc. equivalent to 0.5% BSA or greater. The free chlorine concentration (as Cl = 35.45) of the chlorous acid aqueous solution in the presence of an organic matter including a contaminant, etc. equivalent to 0.5% BSA or greater can be at least 10 mg/L, at least 20 mg/L, at least 30 mg/L, at least 40 mg/L, at least 50 mg/L, at least 60 mg/L, at least 70 mg/L, at least 80 mg/L, at least 90 mg/L, at least 100 mg/L, at least 200 mg/L, at least 300 mg/L, at least 400 mg/L, at least 500 mg/L, at least 600 mg/L, at least 700 mg/L, at least 800 mg/L, at least 900 mg/L, at least 1000 mg/L, at least 2000 mg/L, at least 3000 mg/L, at least 4000 mg/L, at least 5000 mg/L, at least 6000 mg/L, at least 7000 mg/L, at least 8000 mg/L, at least 9000 mg/L, or at least 10000 mg/L.

In the present disclosure, a specification value of the chlorous acid aqueous solution can be 4 to 6%.

In the present disclosure, the coronavirus can be a Letovirinae or Orthocoronavirinae virus. In the present disclosure, the coronavirus can be a virus of the genus alphacoronavirus, betacoronavirus, gammacoronavirus, or deltacoronavirus. In the present disclosure, the coronavirus can be a virus of the genus betacoronavirus.

In the present disclosure, the coronavirus can be a virus of the subgenus Colacovirus, Decacovirus, Duvinacovirus, Luchacovirus, Minacovirus, Minunacovirus, Myotacovirus, Nyctacovirus, Pedacovirus, Rhinacovirus, Setracovirus, Soracovirus, Sunacovirus, Tegacovirus, Embecovirus, Hibecovirus, Merbecovirus, Nobecovirus, Sarbecovirus, Andecovirus, Buldecovirus, Herdecovirus, Brangacovirus, Cegacovirus, or Igacovirus.

In the present disclosure, the coronavirus can be a coronavirus that infects a human.

In the present disclosure, the coronavirus can be HCoV-HKU1, HCoV-OC43, SARS coronavirus (SARS-CoV), MERS coronavirus (MERS-CoV), or 2019 novel coronavirus (SARS-CoV-2) .

In the present disclosure, the coronavirus can be SARS coronavirus (SARS-CoV), MERS coronavirus (MERS-CoV), or 2019 novel coronavirus (SARS-CoV-2).

In the present disclosure, the killing agent can be a killing agent for hands and fingers.

The present disclosure also provides a method of killing a coronavirus by using a chlorous acid aqueous solution.

In the present disclosure, the coronavirus is contacted with the chlorous acid aqueous solution in the absence of an organic matter.

In the present disclosure, a free chlorine concentration (as Cl = 35.45) of the chlorous acid aqueous solution is at least 1 mg/L. The free chlorine concentration (as Cl = 35.45) of the chlorous acid aqueous solution in the absence of an organic matter can be at least 1 mg/L, at least 2 mg/L, at least 3 mg/L, at least 4 mg/L, at least 5 mg/L, at least 6 mg/L, at least 7 mg/L, at least 8 mg/L, at least 9 mg/L, at least 10 mg/L, at least 15 mg/L, at least 20 mg/L, at least 25 mg/L, at least 30 mg/L, at least 35 mg/L, at least 40 mg/L, at least 45 mg/L, at least 50 mg/L, at least 60 mg/L, at least 70 mg/L, at least 80 mg/L, at least 90 mg/L, at least 100 mg/L, at least 110 mg/L, at least 120 mg/L, at least 130 mg/L, at least 140 mg/L, at least 150 mg/L, at least 160 mg/L, at least 170 mg/L, at least 180 mg/L, at least 190 mg/L, or at least 200 mg/L.

In the present disclosure, the coronavirus is contacted with the chlorous acid aqueous solution in the presence of an organic matter.

In the present disclosure, the free chlorine concentration (as Cl = 35.45) of the chlorous acid aqueous solution can be at least 10 mg/L, at least 20 mg/L, at least 30 mg/L, at least 40 mg/L, at least 50 mg/L, at least 60 mg/L, at least 70 mg/L, at least 80 mg/L, at least 90 mg/L, at least 100 mg/L, at least 200 mg/L, at least 300 mg/L, at least 400 mg/L, at least 500 mg/L, at least 600 mg/L, at least 700 mg/L, at least 800 mg/L, at least 900 mg/L, at least 1000 mg/L, at least 2000 mg/L, at least 3000 mg/L, at least 4000 mg/L, at least 5000 mg/L, at least 6000 mg/L, at least 7000 mg/L, at least 8000 mg/L, at least 9000 mg/L, or at least 10000 mg/L.

The present disclosure also provides a chlorous acid aqueous solution for killing a coronavirus.

### (Chlorous acid aqueous solution and manufacturing example thereof)

The chlorous acid aqueous solution used in the present disclosure has the features found by the inventors. A chlorous acid aqueous solution manufactured by any method, such as a known manufacturing method described in a reference described above, can be used. Examples of typical composition that can be used include, but are not limited to, a combination of 61.40% chlorous acid aqueous solution, 1.00% potassium dihydrogen phosphate, 0.10% potassium hydroxide, and 37.50% purified water (sold by the Applicant; 72% chlorous acid aqueous solution corresponds to 30000 ppm of chlorous acid). This agent reduces the decrease in chlorous acid due to contact with an organic matter under an acidic condition, but maintains the sterilizing effect. The agent also has a feature of having minor chlorine gas generation, which suppresses amplification of odor from the mixture of chlorine and organic matter.

In one embodiment, the chlorous acid aqueous solution of the present disclosure can be produced by adding and reacting sulfuric acid or an aqueous solution thereof to an aqueous sodium chlorate solution at an amount and concentration where the pH value of the aqueous solution can be maintained at 3.4 or lower to generate chloric acid, and subsequently adding hydrogen peroxide in an amount equivalent to or greater than the amount required for a reduction reaction of the chloric acid.

Further, in another embodiment, the chlorous acid aqueous solution of the present disclosure can be produced by adding one inorganic acid or inorganic acid salt, two or more types thereof, or a combination thereof to an aqueous solution prepared from producing chlorous acid by adding and reacting sulfuric acid or an aqueous solution thereof to an aqueous sodium chlorate solution at an amount and concentration where the pH value of the aqueous solution can be maintained at 3.4 or lower to generate chloric acid, and subsequently adding hydrogen peroxide in an amount equivalent to or greater than the amount required for a reduction reaction of the chloric acid, and adjusting the pH value within the range from 2.3 to 8.5.

Furthermore, in another embodiment, the chlorous acid aqueous solution of the present disclosure can be produced by adding one inorganic acid, inorganic acid salt, organic acid, or organic acid salt, two or more types thereof, or a combination thereof to an aqueous solution prepared from producing chlorous acid by adding and reacting sulfuric acid or an aqueous solution thereof to an aqueous sodium chlorate solution at an amount and concentration where the pH value of the aqueous solution can be maintained at 3.4 or lower to generate chloric acid, and subsequently adding hydrogen peroxide in an amount equivalent to or greater than the amount required for a reduction reaction of the chloric acid, and adjusting the pH value within the range from 2.3 to 8.5.

Further still, in another embodiment, the chlorous acid aqueous solution of the present disclosure can be produced from adding one inorganic acid, inorganic acid salt, organic acid, or organic acid salt, two or more types thereof, or a combination thereof after adding one inorganic acid or inorganic acid salt, two or more types thereof, or a combination thereof to an aqueous solution prepared from producing chlorous acid by adding and reacting sulfuric acid or an aqueous solution thereof to an aqueous sodium chlorate solution at an amount and concentration where the pH value of the aqueous solution can be maintained at 3.4 or lower to generate chloric acid, and subsequently adding hydrogen peroxide in an amount equivalent to or greater than the amount required for a reduction reaction of the chloric acid, and adjusting the pH value within the range from 2.3 to 8.5.

Further, in another embodiment, carbonic acid, phosphoric acid, boric acid, or sulfuric acid can be used as the inorganic acid in the method described above.

Further still, in another embodiment, carbonate, inorganic hydroxide, phosphate, or borate can be used as the inorganic acid salt.

Further, in another embodiment, sodium carbonate, potassium carbonate, sodium bicarbonate, or potassium bicarbonate can be used as the carbonate.

Furthermore, in another embodiment, sodium hydroxide, potassium hydroxide, calcium hydroxide, or barium hydroxide can be used as the inorganic hydroxide.

Further still, in another embodiment, disodium hydrogen phosphate, sodium dihydrogen phosphate, trisodium phosphate, tripotassium phosphate, dipotassium hydrogen phosphate, or potassium dihydrogen phosphate can be used as the phosphate.

Further, in another embodiment, sodium borate or potassium borate can be used as the borate.

Furthermore, in another embodiment, succinic acid, citric acid, malic acid, acetic acid, or lactic acid can be used as the organic acid.

Further still, in another embodiment, sodium succinate, potassium succinate, sodium citrate, potassium citrate, sodium malate, potassium malate, sodium acetate, potassium acetate, sodium lactate, potassium lactate, or calcium lactate can be used as the organic acid salt.

In a method of manufacturing an aqueous solution comprising chlorous acid (HClO₂) (chlorous acid aqueous solution) that can be used as a bacteria-killing agent, chlorous acid (HClO₂) is produced by adding hydrogen peroxide (H₂O₂) in an amount required to produce chlorous acid by a reducing reaction of chloric acid (HClO₃) obtained by adding sulfuric acid (H₂SO₄) or an aqueous solution thereof to an aqueous solution of sodium chlorate (NaClO₃) so that the aqueous solution is in an acidic condition. The basic chemical reaction of this method of manufacturing is represented by the following formula A and formula B.
[Chemical Formula 1]

2NaClO₈+H₂SO₄ **→** 2HClO₃+Na₂SO₄ ↓ (formula A)

HClO₈+H₂O₂ → HClO₂+H₂O+O₂ ↑ (formula B)

Formula A indicates that chloric acid is obtained by adding sulfuric acid (H₂SO₄) or an aqueous solution thereof at an amount and concentration where the pH value of an aqueous sodium chlorate (NaClO₃) solution can remain acidic. Next, formula B indicates that chloric acid (HClO₃) is reduced by hydrogen peroxide (H₂O₂) to produce chlorous acid (HClO₂).
[Chemical Formula 2]

HClO₃+H₂O₂ → 2ClO₂+H₂O+O₂ ↑ (formula C)

2 ClO₂+H₂O₂ → 2HClO₂+O₂ ↑ (formula D)

2 ClO₂+H₂O ⇔ HClO₂+HClO₃ (formula E)

2 HClO₂⇔H₂O+Cl₂O₃ (formula F)

At this time, chlorine dioxide gas (ClO₂) is generated (formula C). However, coexistence with hydrogen peroxide (H₂O₂) produces chlorous acid (HClO₂) through the reactions in formulas D to F.

Meanwhile, the produced chlorous acid (HClO₂) has a property of being decomposed early into chlorine dioxide gas or chlorine gas due to the presence of chloride ion (Cl⁻), hypochlorous acid (HClO), and other reduction product, or resulting in a plurality of chlorous acid molecules reacting to decompose one another. Thus, it is necessary to prepare chlorous acid (HClO₂) so that the state of chlorous acid can be sustained for an extended period of time in order to be useful as a bacteria killing agent.

In this regard, chlorous acid (HClO₂) can be stably sustained over an extended period of time from creating a transition state to delay a decomposition reaction by adding one inorganic acid, inorganic acid salt, organic acid, or organic acid salt, two or more types thereof, or a combination thereof to the chlorous acid (HClO₂) or chlorine dioxide gas (ClO₂) obtained by the method described above or an aqueous solution containing them.

In one embodiment, it is possible to utilize a mixture prepared by adding an inorganic acid or inorganic acid salt, specifically carbonate or inorganic hydroxide, two or more types thereof, or a combination thereof to the chlorous acid (HClO₂) or chlorine dioxide gas (ClO₂) obtained by the method described above or an aqueous solution containing them.

In another embodiment, it is possible to utilize a mixture prepared from adding one inorganic acid, inorganic acid salt, organic acid, or organic acid salt, two or more types thereof, or a combination thereof to an aqueous solution to which one inorganic acid or inorganic acid salt, specifically carbonate or inorganic hydroxide, two or more types thereof, or a combination thereof is added.

Additionally, in another embodiment, it is possible to utilize a mixture prepared by adding one inorganic acid, inorganic acid salt, organic acid, or organic acid salt, two or more types thereof, or a combination thereof to an aqueous solution manufactured by the method described above.

Examples of the inorganic acid described above include carbonic acid, phosphoric acid, boric acid, and sulfuric acid. Examples of the inorganic acid salt include phosphate and borate, in addition to carbonate and inorganic hydroxide. Specifically, sodium carbonate, potassium carbonate, sodium bicarbonate, or potassium bicarbonate may be used as the carbonate; sodium hydroxide, potassium hydroxide, calcium hydroxide, or barium hydroxide may be used as the inorganic hydroxide; disodium hydrogen phosphate, sodium dihydrogen phosphate, trisodium phosphate, tripotassium phosphate, dipotassium hydrogen phosphate, or potassium dihydrogen phosphate may be used as the phosphate; and sodium borate or potassium borate may be used as the borate. Examples of the organic acid described above include succinic acid, citric acid, malic acid, acetic acid, and lactic acid. Further, sodium succinate, potassium succinate, sodium citrate, potassium citrate, sodium malate, potassium malate, sodium acetate, potassium acetate, sodium lactate, potassium lactate, or calcium lactate is suitable as the organic acid salt.

When an acid and/or a salt thereof is added, a transition state, such as Na⁺ + ClO₂⁻ < - > Na-ClO₂, K⁺ + ClO₂⁻ < - > K-ClO₂, or H⁺ + ClO₂⁻ < - > H-ClO₂ can be temporarily created to delay the progression of chlorous acid (HClO₂) to chlorine dioxide (ClO₂), which enables the manufacture of an aqueous solution comprising chlorous acid (HClO₂) that sustains chlorous acid (HClO₂) for an extended period of time and generates a low amount of chlorine dioxide (ClO₂).

The following represents the decomposition of chlorite in an acidic solution.
[Chemical Formula 3]

5ClO₂⁻ + 4H⁺ → 4ClO₂ + 5Cl⁻ + 2H₂O (5NaClO₂ + 4CH₃COOH → 4ClO₂ + 4CH₃COONa + NaCl + 2H₂O) (a)

3ClO₂⁻ → 2ClO₃⁻ + Cl⁻ (b)

(3NaClO₂ → 2NaClO₃ + NaCl) autodegradation ClO₂⁻ → Cl⁻ + 2O (c)

As expressed in the formula, the rate of decomposition of an aqueous chlorite solution is greater at lower pH, i.e., more acidic. Specifically, the absolute rates of reactions (a), (b), and (c) in the formula described above increase. For example, although the ratio accounted for by reaction (a) decreases at a lower pH, the total decomposition ratio changes significantly, i.e., to a larger value. Thus, the amount of generated chlorine dioxide (ClO₂) also increases as the pH decreases. Therefore, a lower pH value results in sooner sterilization or bleaching. However, irritable and harmful chlorine dioxide gas (ClO₂) renders an operation more difficult and adversely affects the health of a human being. Further, a reaction of chlorous acid to chlorine dioxide progresses quicker to render chlorous acid unstable. In addition, the period during which a sterilizing power can be sustained is very short.

When the inorganic acids, inorganic acid salts, organic acids, or organic acid salts described above are added to an aqueous solution comprising chlorous acid (HClO₂), pH values are adjusted in the range of 2.3 to 8.5 from the viewpoint of balancing suppression of the generation of chlorine dioxide and sterilizing power.

The chlorous acid aqueous solution of the present disclosure can also be an aqueous solution obtained by adding sulfuric acid to an aqueous solution obtained by electrolysis of a solution prepared by adding hydrochloric acid to a saturated sodium chloride solution under acidic conditions in a diaphragm-free electrolytic tank (referred to those comprised of anode and cathode that are not separated by a diaphragm) to make the aqueous solution strongly acidic, and adding a hydrogen peroxide solution for a reaction with chloric acid generated thereby. A chlorous acid aqueous solution can be those described in Japan's Specifications and Standards for Food Additives, 9th Edition 2018 (Ministry of Health, Labour and Welfare; Consumer Affairs Agency).

Examples of chlorous acid aqueous solution formulations that can be used in the present disclosure include HonbuSankei's "Kare for Hands", "Rare for Hands Pro-free", "Rare for Fresh", "Autoloc Super", "New Autoloc SP", "Rare for peace Pro-free", "Rare for No. 3", "Rare for Norobarrier Plus", "KlorusKare 8", "KlorusKare 10", etc.

### (Problems in comparing/evaluating antimicrobial effects of chlorous acid aqueous solutions and sodium hypochlorite)

Comparison/evaluation of antimicrobial effects of chlorous acid aqueous solutions and sodium hypochlorite is problematic in that the concentration of chlorine oxide can be denoted in terms of available chlorine concentration or free chlorine, while antimicrobial effects are dependent on free chlorine, which is the source of oxidation power. In addition, sodium hypochlorite has a near 1:1 relationship between free chlorine and available chlorine concentration, but the available chlorine concentration and free chlorine do not necessarily match for chlorous acid aqueous solutions in the same manner as sodium hypochlorite. For this reason, it is necessary to compare sterilizing powers of both agents on a level field by using oxidation power that represents the antimicrobial effect, i.e., free chlorine, instead of available chlorine concentrations.

Oxidation power of a chlorine oxide agent is generally found through a measurement method utilizing colorimetry such as a TMB method or DPD method. However, there is no standard for measuring free chlorine in a chlorous acid aqueous solution in the same manner as sodium hypochlorite. For this reason, calibration curves are created by using 1 mg/L of free chlorine (as Cl = 35.45) of sodium hypochlorite as 1 oxidation power. Oxidation power can be represented by free chlorine (as Cl = 35.45). For comparison by the same free chlorine level, free chlorine (as Cl = 35.45) of sodium hypochlorite is generated from Cl radicals, but free chlorine of chlorous acid aqueous solutions has an HClO₂ as the source of generation. Thus, it is difficult to compare when evaluating with respect to a standard in accordance therewith. Therefore, a chlorous acid aqueous solution is compared and evaluated with sodium hypochlorite on a level field by using the same standard through calculation at oxidation power 1 = free chlorine (as Cl = 35.45) of 1 mg/L in the same manner as sodium hypochlorite.

As a measuring method of free chlorine (as Cl = 35.45), a buffer and a DPD indicator are added to a sample and a wavelength of 510 nm is measured with an absorption spectrophotometer, and for measurement of free chlorine (as Cl = 35.45) in the presence of an organic matter, measurement is taken at a wavelength of 655 nm using a TMB reagent to deduce the concentration from the measurement values. Further, a method of conducting a test for studying the sterilizing effect includes preparing free chlorine (as Cl = 35.45) of a test agent by a DPD method, contacting each agent with an organic matter-containing bacterial solution, neutralizing the solution with sodium thiosulfate after a certain period of time has passed, and checking the viable bacteria count in the neutral solution. The available chlorine content can be found by using an iodometric method

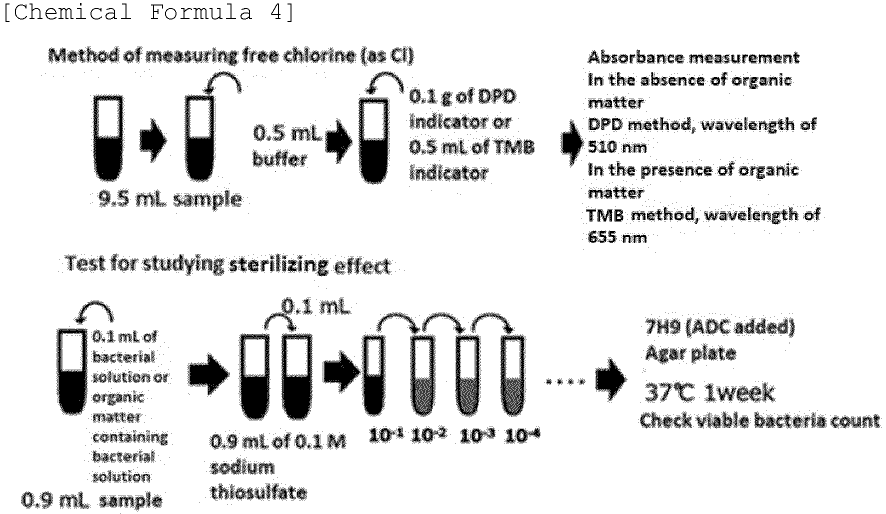

### (Method of use of chlorous acid aqueous solution in coronavirus disease 2019 (COVID-19))

Coronavirus disease 2019 (COVID-19) is a respiratory infection caused by SARS-CoV-2 (COVID-19 virus). The COVID-19 virus is transmitted mainly through close physical contact and respiratory droplets, while airborne transmission is possible during aerosol-generating medical procedures. At this time, transmission of the COVID-19 virus had not been conclusively linked to contaminated environmental surfaces (surfaces of facilities, equipment, objects, etc.) in studies. However, such a link has been informed by evidence of contamination of equipment, objects, etc. in healthcare settings and environmental surface contamination that was linked to subsequent infection transmission in other coronaviruses. Therefore, the objective is to reduce any role that fomites might play in the transmission of COVID-19 in healthcare and non-healthcare settings.

Environmental surfaces in healthcare settings include furniture and other fixed items inside and outside of patient rooms and bathrooms, such as tables, chairs, walls, light switches, computer peripherals, electronic equipment, sinks, toilets as well as non-critical (medical) equipment, such as blood pressure cuffs, stethoscopes, wheelchairs and incubators, etc. In non-healthcare settings, environmental surfaces include sinks, toilets, electronics (touch screens and controls), furniture and other fixed items, such as countertops, stairway rails, floors, and walls

Environmental surfaces are more likely to be contaminated with COVID-19 in healthcare settings where medical procedures are performed. Therefore, these surfaces, especially where patients with COVID-19 infections are being cared for, must be properly cleaned and disinfected to prevent further transmission. Similarly, this applies to alternative settings for isolation of persons with COVID-19 infections experiencing uncomplicated and mild illness, including households and non-traditional facilities.

Transmission of the COVID-19 virus has been linked to close contact within closed settings, such as households, health facilities, assisted living and residential institutions. In addition, community settings outside of healthcare settings have been found vulnerable to COVID-19 transmission events including publicly accessible buildings, faith-based community centers, markets, transportation, and business settings. Although the precise role of fomite transmission and necessity for disinfection practices outside of healthcare environments is currently unknown, mitigation of the spread of viral infections, including cleaning and disinfection practices, is needed so that they can be applied in non-healthcare setting environments (for example, current WHO interim guidance for non-healthcare setting environments, including environmental cleaning and disinfection recommendations, is directed to faith-based community settings, funerary services, workplaces, food sector, accommodation sector, aviation sector, maritime sector, schools, prisons, and other places of detention). In all settings, including those where cleaning and disinfection are not possible on a regular basis due to resource limitations, frequent hand washing and avoiding touching the face should be the primary prevention approaches to reduce any potential transmission associated with surface contamination.

Like other coronaviruses, SARS-CoV-2 is an enveloped virus with a fragile outer lipid envelope that makes it more susceptible to disinfectants compared to non-enveloped viruses such as rotavirus, norovirus, and poliovirus. Studies have evaluated the persistence of the COVID-19 virus infectivity on different surfaces. One study found that the COVID-19 virus remained viable for 1 day on cloth and wood surfaces, for 2 days on glass surfaces, for 4 days on stainless steel and plastic surfaces, and for 7 days on the outer layer of a medical mask. Another study found that the COVID-19 virus survived 4 hours on copper surfaces, 24 hours on cardboard surfaces, and 72 hours on plastic and stainless steel surfaces. The COVID-19 virus also survives in a wide range of pH values and ambient temperatures, but is susceptible to heat and standard disinfection methods. These studies, however, were conducted under laboratory conditions in the absence of cleaning and disinfection practices and should be interpreted with caution for implementation in the real-world environment.

The present disclosure provides cleaning and disinfection of environmental surfaces in the context of coronavirus disease 2019 (COVID-19).

The present disclosure can be targeted for healthcare professionals, public health professionals, and health authorities that are developing and implementing policies and standard operating procedures (SOP) on the cleaning and disinfection of environmental surfaces in the context of COVID-19. Comprehensive guidance for cleaning and disinfecting an environment is described in "Essential environmental health standards in health care" published by WHO and "Best practices for environmental cleaning in healthcare facilities in resource-limited settings" jointly published by US Centers for Disease Control and Prevention and Infection Control Africa Network. The procedures for decontamination of instruments and semi-critical and critical medical devices are described in "Decontamination and reprocessing of medical devices for health-care facilities" published by WHO.

### Principles of cleaning and disinfection

Cleaning helps to remove or significantly reduce viruses on contaminated environmental surfaces and is an essential first step in any disinfection process. Cleaning with water, soap (or a neutral detergent), and mechanical action (brushing or scrubbing) removes and reduces dirt, debris, and other organic matter such as blood, secretions, and excretions, but does not kill microorganisms. Organic matter can impede direct contact of a disinfectant to a surface and inactivate the antiviral properties or mode of action of disinfectants. Therefore, a chemical disinfectant, such as chlorine or alcohol, should be applied after cleaning to kill any remaining microorganisms.

Disinfectant solutions are prepared and used according to the manufacturer's recommendations for volume and contact time. Concentrations with inadequate dilution during preparation (too high or too low) may reduce their effectiveness. High concentrations increase chemical exposure to users and may also damage environmental surfaces. Enough disinfectant solution should be applied to allow surfaces to remain wet and untouched long enough for the disinfectant to inactivate viruses, as recommended by the manufacturer. Cleaning of training environment in healthcare settings is a complex infection prevention and control intervention that requires a multipronged approach, which may include training, monitoring, auditing and feedback, reminders, and displaying SOPs in key areas.

Training for cleaning staff should be based on the policies and SOPs of the healthcare facility and national guidelines. It should be structured, targeted, and delivered in the right style (e.g., participatory, at the appropriate literacy level), and it should be mandatory during staff induction to a new workplace. The training program should include instructions on risk assessment and ensure safe disinfectant preparation, mechanical cleaning and equipment use, standard precautions, and transmission-based precautions. Refresher courses are recommended to encourage good practice. In healthcare facilities and public buildings, posters or other guidance should be visible to cleaning workers and others to guide and remind them about the proper procedures on disinfectant preparation and use.

### Cleaning and disinfection techniques and required supplies

Cleaning should progress from the least soiled (cleanest) to the most soiled (dirtiest) areas, and from the higher to lower levels so that debris may fall on the floor and is cleaned last in a systematic manner to avoid missing any areas. Use fresh cloths at each cleaning session (e.g., routine daily cleaning in a general inpatient ward). Discard cloths that are no longer saturated with solution. For areas considered to be at high risk of COVID-19 virus contamination, use a new cloth to clean each patient's bed. Soiled cloths should be reprocessed properly after each use and an SOP should be available for the frequency of changing cloths.

Cleaning equipment (e.g. buckets) should be well maintained. Equipment used for isolation areas for patients with COVID-19 should be color-coded and separated from other equipment. Detergent or disinfectant solutions become contaminated during cleaning and progressively less effective if the organic load is too high; therefore, the continued use of the same solution may transfer the microorganisms to the subsequent surfaces. Thus, detergent and/or disinfectant solutions must be discarded after each use in areas with suspected/confirmed patients with COVID-19. It is recommended that fresh solution be prepared on a daily basis or for each cleaning shift. Buckets should be washed with detergent, rinsed, dried, and stored inverted to drain fully when not in use.

### Products for cleaning and disinfection

Follow the manufacturer's instructions so that disinfectants are prepared and handled safely, wearing the appropriate personal protective equipment (PPE) to avoid chemical exposure. The selection of disinfectants should take account of the microorganisms targeted, as well as the recommended concentration and contact time, the compatibility of the chemical disinfectants and surfaces, toxicity, ease of use, stability of the product, etc. The selection of disinfectants should meet local authorities' requirements for market approval, including any regulations applicable to specific sectors, such as the healthcare and food industries.

### Cleaning and disinfection in healthcare settings

Environmental cleaning and disinfection in clinical, non-traditional facilities and home-based healthcare setting should follow detailed SOPs with a clear delineation of responsibilities (e.g. housekeeping or clinical staff), regarding the type of surfaces and frequency of cleaning. Particular attention should be paid to environmental cleaning of high-touch surfaces, such as light switches, bed rails, door handles, intravenous pumps, tables, water/beverage pitchers, trays, mobile cart rails, and sinks, which should be performed frequently. However, all touchable surfaces should be disinfected. Cleaning practices and cleanliness should be routinely monitored. The number of cleaning staff should be planned to optimize cleaning practices. Health workers should be made aware of cleaning schedules and cleaning completion times to make informed risk assessments when performing touch contact with surfaces and equipment, to avoid contaminating hands and equipment during patient care.

### Cleaning and disinfection in non-healthcare settings

There is no evidence for equating the risk of fomite transmission of the COVID-19 virus in the healthcare settings to non-healthcare settings. However, it is still important to reduce potential for COVID-19 virus contamination in non-healthcare settings, such as in the home, office, schools, gyms, or restaurants. High-touch surfaces in these non-healthcare settings should be identified for priority disinfection. Specifically, these include door and window handles, kitchen and food preparation areas, countertops, bathroom surfaces, toilets and taps, touchscreen personal devices, personal computer keyboards, work surfaces, etc. The disinfectant and its concentration should be carefully selected to avoid damaging surfaces and to avoid or minimize toxic effects on household members or users of public spaces. The environmental cleaning techniques and cleaning principles should be followed as far as possible. Surfaces should always be cleaned with soap and water or a detergent to remove organic matter, followed by disinfection.

### Personal safety when preparing and using disinfectants

Cleaners should wear adequate personal protective equipment (PPE) and be trained to use it safely. When working in places used by suspected or confirmed COVID-19 patients, or where screening, triage, and clinical consultations are carried out, cleaners should wear the following PPE: gown, heavy duty gloves, medical mask, eye protection (in the presence of a risk of splash from organic material or chemicals), and boots or closed work shoes. Disinfectant solutions should always be prepared in well-ventilated areas. Avoid combining disinfectants, both during preparation and usage, as such mixtures cause respiratory irritation and can release potentially fatal gases, in particular when combined with hypochlorite solutions. Personnel preparing or using disinfectants in healthcare settings require specific PPE, due to the high concentration of disinfectants used in these facilities and the longer exposure time to the disinfectants during the workday. Thus, PPE required for preparing or using disinfectants in healthcare settings includes uniforms with long sleeves, closed work shoes, gowns and/or impermeable aprons, rubber gloves, medical mask, and eye protection (preferably face shield). In non-healthcare settings, resource limitations permitting, where disinfectants are being prepared and used, the minimum recommended PPE is rubber gloves, impermeable aprons, and closed shoes. Eye protection and medical masks may also be needed to protect against chemicals if there is a risk of splashing.

### (Comparison of chlorous acid aqueous solution with other sterilizing agents)

Sodium hypochlorite and hypochlorous acid water are reported to have no virucidal effect unless the free chlorine concentration is 35 ppm or higher which is unachievable with electrolyzed hypochlorous acid water and organic matters are thoroughly removed. pH adjusted sodium hypochlorite, which is not approved as a food additive, is referred to as a "high concentration hypochlorous acid water" and is subjected to evaluation. Hypochlorous acid water itself is not demonstrated to have an activation effect.

An effect of inactivating novel coronaviruses of hypochlorous acid water cannot be found under conditions other than limited conditions, i.e., in the absence of an organic matter. Meanwhile, it is known that a chlorous acid aqueous solution can, in the presence of an organic matter, achieve at least a 99.9% inactivation effect even with a 8x diluent. A chlorous acid aqueous solution can, in the absence of an organic matter, achieve a complete inactivation effect on novel coronaviruses even with a 20x diluent.

An inactivation effect of alcohol cannot be expected on enveloped viruses unless the concentration is 70% (v/v) or greater. Such a high concentration of alcohol would also remove oil and fat of hands and fingers of a user upon use. Moreover, measures that prohibit access to a facility unless an alcohol formulation placed at an entrance of a public institution is used resulted in severely chapped hands of users, which has already developed into a significant social issue.

This is described in more detail herein.

### 1. Sodium hypochlorite: food additive sterilizer.

Since a "sodium hypochlorite solution" is prepared by blowing chlorine gas into sodium hydroxide (caustic soda), such a solution that is strongly alkaline with an available chlorine concentration of 4% (40,000 ppm) to 12% (120,000 ppm) is commercially sold and used by diluting the solution in accordance with the objective.

Sodium hypochlorite solutions exhibit broad sterilizing activity on bacteria, fungi, viruses, etc. and are used to the extent such that the solutions can be considered as a standard disinfectant for antiviral measures in Japan.

In general, the solution is used at a concentration of 200 ppm or higher for wiping or immersion for sterilization, microbe removal, disinfection, etc. when cleaning door knobs, cooking utensils and equipment, food preparation facilities, kitchens, or other common facilities as an antiviral measure.

As measures for viruses without an envelope, use at 500 ppm or higher is recommended. 1000 ppm or higher and in some cases 5000 ppm is required for the treatment of feces, vomits, etc., with a large number of viruses and organic matters. Since the solution has a mechanism of sterilization through denaturation of proteins or lipids at a highly alkaline region, the solution is highly irritable to the skin or mucous membrane and damages the hand and skin. Thus, caution is required for the handling thereof.

One of the reasons that the use thereof is avoided in practice is not only due to the irritating odor upon treatment, but also the severity of residual odor after treatment.

### 2. Hypochlorous acid water

Regular hypochlorous acid water is described in Japan's Specifications and Standards for Food Additives, 9th Edition. "Hypochlorous acid water" is a new nomenclature specified by the country upon designating acidic electrolyzed water as a food additive sterilizer as a set with a dedicated generator (electrolyzer). When diluted saline or hydrochloric acid water is electrolyzed with an electrolyzer, a chloride ion (Cl⁻) is generated by an anodic reaction and reacts with a water molecule (H₂O) to generate acidic electrolyzed water comprising hypochlorous acid (HClO) and hydrochloric acid (HCl). This is the true "hypochlorous acid water" and is the reason it was originally known as acidic electrolyzed water. Such "hypochlorous acid water" is characterized by a low concentration but high activity, exhibiting activity against wide-ranging bacteria, fungi, and viruses, and high level of safety, but reacting immediately with an organic matter in the presence of an organic matter to lose activity significantly. Thus, it is important to use hypochlorous acid water after removing organic matter, i.e., contamination, in advance. Such "hypochlorous acid water" itself is not commercially available. A product produced by a user purchasing a generator and operating the generator at the site of use should be referred to as "hypochlorous acid water". A regulation generator (JIS specification: JISB-8701 was established in 2017) is set to always generate "hypochlorous acid water" with available chlorine of 10 ppm to 80 ppm at the usage concentration. Hypochlorous acid water is in principle used as flowing water, i.e., direct flow, or overflow without dilution while still as fresh as possible immediately after generation. Since hypochlorous acid water is continuously generated from a flowing generator connected to a tap water faucet, flowing hypochlorous acid water can be used, or hypochlorous acid water can be stored in a large tank and used as flowing hypochlorous acid water therefrom through a pipe.

Currently, three types, i.e., "strongly acidic (pH of 2.7 or less), weakly acidic (pH of 2.7 to 5.0), and slightly acidic (pH of 5.0 to 6.5)", of acidic electrolyzed water are approved as "hypochlorous acid water". The available chlorine concentrations are 20 to 60 ppm for strongly acidic, 10 to 60 ppm for weakly acidic, and 10 to 80 ppm for slightly acidic electrolyzed water. Accordingly, viewed as a whole, acidic electrolyzed water has a pH of 6.5 or less and available chlorine concentration of 10 ppm to 80 ppm.

### [Table 1]

**Table 1. Properties of sterilizer, disinfecting agent, etc. used in measures for novel coronavirus**

| | **Sodium hypochlorite** | **Hypochlorous acid water** | **Pseudo-hypochlorous acid water** | **Chlorous acid aqueous solution** | **Disinfecting ethanol** | |
|---|---|---|---|---|---|---|
| **Status of approval** | **Food additive sterilizer** | **Food additive sterilizer** | **None in particular** | **Food additive sterilizer** | **Disinfecting agent/food additive** | |
| **Safety** | **Undiluted solution is hazardous** | **Highly safe** | **Unknown (caution required for high concentration )** | **Safe (at usage concentration)** | **Flammable** | |
| **Manufacturing** | ***Electrolysis of** | **Electrolysis of** | **Dilute sodium** | **Add sulfuric acid** | **Admix purified** | |
| **method** | | **saturated saline *Cl₂ injection into NaOH** | **HCl or NaCl** | **hypochlorite by admixing acid** | **and hydrogen peroxide to electrolyte from electrolysis of saturated saline** | **water with ethanol** |
| **Undiluted solution concentration** | | **4 to 12%** | **10 to 80 ppm** | **No specification** | **4 to 6%** | **76.9 to 81.4%** |
| **Usage concentration** | | **100 to 5000 ppm** | **10 to 80 ppm** | **No specification** | **200 ppm to 8000 ppm** | **76.9 to 81.4%** |
| **Physical property (pH)** | | **>7.5 (alkaline)** | **6.5 or less (acidic)** | **Acidic (no specification)** | **Weakly acidic to neutral** | **Neutral** |
| **Primary component** | | **Hypochlorite ion** | **Hypochlorous acid** | **Hypochlorous acid** | **Chlorous acid** | **Ethanol** |
| **Basic usage method** | | **Dilution/ immersion** | **Cleaning with flowing solution** | **Immersion** | **Dilution/immersion /spray/wipe** | **Spray/scraping** |
| **Portability Subject of use** | | **○** | **x** | **?** | **○** | **○** |
| | **Food** | **Δ** | **○** | **x** | **○** | **x** |
| | **Metal** | **Δ** | **Δ** | **Δ** | **Δ** | **○** |
| | **Non-metal** | **○** | **○** | **Δ** | **○** | **○** |
| | **Environment** | **Δ** | **○** | **?** | **○** | **○** |
| | **Skin** | **x** | **○** | **x** | **○** | **Δ (chapped hands)** |
| | **Mucous membrane** | **x** | **○** | **x** | **○** | **x** |
| | **Antibacterial/ fungal activity** | **Broad range** | **Broad range** | **?** | **Broad range** | **Broad range except for endospore** |
| | **Antiviral activity** | | | | | |
| | **Influenza** | **○** | **⊚** | **?** | **⊚** | **○** |
| | **Norovirus (alternative)** | **○** | **⊚** | **?** | **⊚** | **∇** |
| | **Novel coronavirus** | **○** | **⊚** | **?** | **⊚** | **○** |
| | **Subject of use** | **○ Usable, Δ Usable (can cause rusting), x Unusable, ? Unknown** | | | | |
| | **Antiviral activity** | **○ Effective, V Low efficacy, ⊚ Effective even in the presence of organic matter** | | | | |

Cited from, and added description of (section of chlorous acid aqueous solution) to, Functional Water Newsletter No. 95 (published on May 7, 2020) published by Functional Water Foundation with permission.

### 3. Pseudo-hypochlorous acid water (not officially approved)

An aqueous solution of "sodium hypochlorite" admixed/ diluted with acid is distributed under the name of "hypochlorous acid water". However, this is "pseudo-hypochlorous acid water", which is similar to but different from food additive "hypochlorous acid water".

The most significant issue is that products with a much higher concentration than the concentration specified for "hypochlorous acid water" are distributed due to the lack of a specification for concentration from the Ministry of Health, Labour and Welfare.

With the spread of coronavirus disease 2019, many inquiries and complaints are filed with regard to health hazards (throat or eye pain, etc.) due to such "pseudo-hypochlorous acid water".

The issue with "pseudo-hypochlorous acid water" is that this is not designated as a food additive so that there are no official specification standards. The lack of objective evidence for safety or regulation for concentrations is also issue. For this reason, pseudo-hypochlorous acid water that is being sold with a high concentration of 200 ppm or higher, and some with a concentration of 1000 ppm, are also distributed.

It is evident that such a product being distributed under the name "hypochlorous acid water" can damage the credibility of actual "hypochlorous acid water".

In this regard, a method of distinction by category names is being contemplated.

It is understood that the name "acidified hypochlorous acid water" has the understanding of the market for the following reason.

"Regarding use of sodium hypochlorite by admixing an acid" in the "Notice of the Chief of Standards Review Division, Food Safety Department, Ministry of Health, Labour and Welfare, No. 0825001 dated August 25, 2004" describes that "2. The so-called electrolyzed water, which is deemed the same as diluted food additive "sodium hypochlorite" in section 2 of "Handing of so-called electrolyzed water" in the Notice of the Chief of the Food Chemistry Division, Pharmaceutical Safety and Environmental Health Bureau, Ministry of Health, Labour, No. 31 dated June 25, 1999, is deemed the same as the above 2. "Hypochlorous acid water". Accordingly, the name "electrolyzed hypochlorous acid water" was established for the "so-called electrolyzed water" after the notice because "hypochlorous acid water" was generally accepted as the common name for a "sodium hypochlorite" diluent.

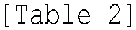

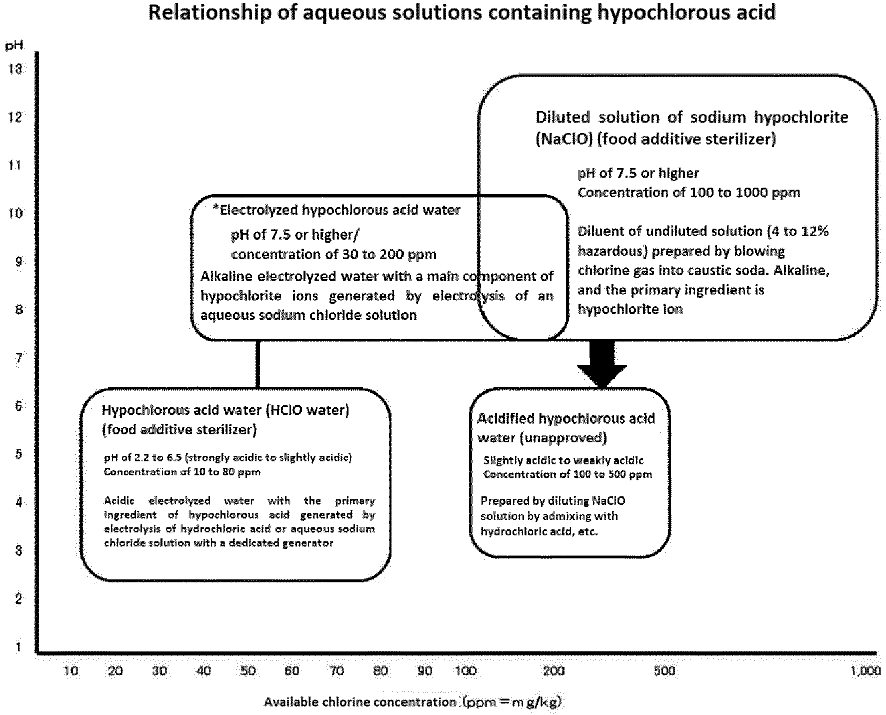

Cited from, and added description (acidified hypochlorous acid water is unapproved) to, Functional Water Newsletter Extra Edition No. R2-1 (published on May 29, 2020) published by Functional Water Foundation with permission.

### 4. Chlorous acid aqueous solution

This is a sterilizer that is the most recently approved as a food additive. The manufacturing method thereof is specified as a definition in Japan's Specifications and Standards for Food Additives, 9th Edition. Many documents report that the primary active ingredient chlorous acid exhibits a potent activity against a broad range of bacteria, fungi, and viruses. Although for an alternative virus, it is reported that "this is the only agent that exhibited a potent inactivation activity, in the abundant presence of organic matters, on noroviruses, which are viruses without an envelope having strong drug resistance" in a 2015 report published by the National Institute of Health Science to enter the limelight. Utility in many settings is expected after the approval as a Class II sterilizing disinfectant in 2018.

"Chlorous acid aqueous solution", which has chlorous acid as a primary active ingredient and successfully stabilized chlorous acid in a solution for an extended period, is distributed in some cases as a 40,000 ppm to 60,000 ppm product [it should be noted that the concentration of "chlorous acid aqueous solution" is represented as content according to an iodometric method (as HClO₂ = 68.46) instead of "chlorous acid content (as HClO₂ = 68.46)", and the oxidation power is indicated as free chlorine concentration according to DPD colorimetric method], just like a "sodium hypochlorite solution", as a raw material for a pharmaceutical product. Meanwhile, chlorous acid aqueous solutions cannot be freely handled. Thus, formulations diluted to 400 ppm or 8000 ppm are distributed as products so that anyone can freely carry and use anytime and anywhere, or use as needed, by utilizing the very high stability in a solution, which is a feature of chlorous acid. This is directly used, or further diluted for use in wiping, immersion, or as droplets. In addition, at low concentrations, metal is hardly corroded with no stimulation to the skin or mucous membrane. Thus, sterilization through spraying (sterilization of food through spraying) is approved. In the US, chlorous acid aqueous solutions are broadly used by not only immersion, but also spraying, mainly in sterilization of pathogenic microorganisms in animal meat or poultry (Campylobacter, Salmonella, enterohemorrhagic Escherichia coli group, etc.), sterilization of resident resistant bacteria, i.e., heat resistant endospores that generally cannot be sterilized by heating, in vegetables, spices, cereals, and legumes, and sterilization of fungi such as mold and yeast. Chlorous acid aqueous solutions have started to appear in the "Manual for Hygiene Management at Large-scale Food Preparation Facilities" and various sanitary codes in Japan.

### 5. Recommended locations of use

As for the method of selecting a sterilizer/disinfectant/microbicide to address viruses, disinfecting alcohol and alcohol formulations are the most suitable to be placed at entrance/exit of buildings and locations with traffic of a large number of people such as public transportation and facilities generally without moisture for microbe removal, sterilization or disinfection of hands and fingers. A certain level of effect is exhibited on bacteria, fungi, and viruses if the alcohol concentration is 50% v/v, or 75% v/v or greater if possible.

However, the drawback of alcohol is the dramatic loss of efficacy upon decrease in the concentration. When the concentration dips below 50% v/v, the efficacy may be dramatically lost. This is due to the sterilization mechanism of alcohol. Since the sterilization mechanism of alcohol is sterilization by denaturation of proteins or lipids through enthalpy of vaporization, this would be effective on viruses with an envelope comprised of proteins or lipids such as influenza viruses and coronaviruses, but less effective on viruses without an envelope such as noroviruses. More than anything, the concentration decreases significantly in environments or locations with a large amount of moisture due to adhering moisture. Thus, alcohol must be considered unsuitable for use at wet areas, locations with a large amount of moisture, or locations where a large amount of water is used. Even if 99% v/v disinfecting alcohol is used for sterilization by spraying 1 cc to 2 cc, the alcohol concentration would be, with the presence of 1 cc to 2 cc of water droplets, 50% or less, resulting in no sterilization effect at all.

This needs to be noted for use.

"Sodium hypochlorite", i.e., bleach, is suitable for sterilization, microbe removal, or disinfection at such locations with a large amount of moisture. Immersion or wiping with a 200 ppm solution of "sodium hypochlorite" is very effective as measures for bacteria, fungi, and viruses. In particular, use of a 500 ppm solution is recommended for measures against non-enveloped viruses. It is understood that it is necessary to treat environments with a large amount of organic matter such as contaminated locations with a 1000 ppm or higher solution, and feces or vomits with a 5000 ppm solution. However, the sterilization mechanism of a "sodium hypochlorite solution" is the same as alcohol and is understood to sterilize by denaturing proteins or lipids at a highly alkaline region. Thus, sodium hypochlorite is rather characterized by being irritable to the skin and mucous membrane and likely to cause chapped skin or hand and damage to the mucous membrane, etc., such that it is very difficult to handle.

Moreover, metal is rapidly corroded, and intense chlorine odor during treatment and post-treatment reaction odor is severe, such that the location cannot be used immediately after sterilization, microbe removal, or disinfection. This is considered a very significant issue as a measure against infections. It is not an exaggeration to say that "hypochlorous acid water" was developed for sterilizing, removing microbes, and disinfecting wide and large locations in its entirety with a large amount of water such as kitchens, food preparation facilities, food processing factories, and central kitchens, regardless of size. Such "hypochlorous acid water" can create a sanitary environment instantly by installing an acidic electrolyzed water generator and washing a facility first with a large amount of water and then rinsing off and sterilizing the entire facility. However, hypochlorous acid water reacts immediately with organic matter or contamination and significantly loses the efficacy under contaminated environments with a large number of organic matter. In such a case, sterilization, microbe removal, and disinfection using "chlorous acid aqueous solution" is recommended. "Chlorous acid aqueous solution" is characterized in that anyone can carry and use a chlorous acid aqueous solution anywhere where it is needed when needed. A liquid product that can be carried around and placed and obtained by electrolysis of salt is only "chlorous acid aqueous solution". Accordingly, chlorous acid aqueous solutions are very suitable to be placed and used in restrooms, washrooms, bathrooms, kitchens, etc. with a large amount of moisture and stains and are very convenient.

In summary, "alcohol" is effective at locations with traffic of a large number of people. However, efficacy is lost at locations with a large amount of moisture, so that "sodium hypochlorite" is effective at locations with a large amount of moisture. However, "sodium hypochlorite" is highly alkaline and is alkaline no matter how much it is diluted, thus resulting in skin or mucous membrane damage and causing chapped skin or hands. In such a case, "hypochlorous acid water" and "chlorous acid aqueous solution" are very effective. "Hypochlorous acid water" is especially recommended for use in kitchens, food preparation facilities, food processing factories, etc. that use a large amount of water. However, such "hypochlorous acid water" requires installation of a generator. Only acidic electrolyzed water that is as fresh as possible generated from the generator exerts an effect. Such "hypochlorous acid water" filled and stored in a bottle cannot be expected to have an effect. It is "chlorous acid aqueous solution" that is the most suitable for use after bottling and storage. In particular, use of such "chlorous acid aqueous solution" is strongly recommended around water such as restrooms, kitchens, bathrooms, and washrooms in a contaminated environment with an especially large amount of organic matter.

Undiluted (99% (v/v)) alcohol is found to have a sterilizing effect (generally 75% or higher). Meanwhile, a sterilizing effect is lost when mixed with water (including when diluted). Accordingly, alcohol treatment should be limited to dry locations and is ineffective in wet areas such as washrooms.

In this regard, a chlorine-based sterilizing agent is generally used instead of alcohol at locations with water. Representative sodium hypochlorite is a strong alkaline agent, and the sterilization effect is from alkaline denaturation of proteins, etc. Since sodium hypochlorite is alkaline no matter how much it is diluted with water, use thereof on a human results in skin or mucous membrane damage including chapped skin. However, hypochlorous acid water and chlorous acid aqueous solution that are near neutral chlorine oxide sterilizing agents have low skin irritability with no concerns for chapped skin. However, hypochlorous acid water is an aqueous solution obtained from an electrolyzer. Hypochlorous acid water discharged from an electrolyzer immediately loses free chlorine, and thus cannot be placed in a bottle and used. Moreover, hypochlorous acid water cannot be carried. In this regard, unlike hypochlorous acid water, a chlorous acid aqueous solution is advantageous over other sterilizing agents in that a chlorous acid aqueous solution can be carried around while maintaining free chlorine. Chlorous acid aqueous solutions in a carryable state are found to have an effect of inactivating novel coronaviruses.

In many cases, acidic sodium hypochlorite prepared by adjusting the pH of sodium hypochlorite is distributed as a "hypochlorous acid water" product, but not all are hypochlorous acid water. Moreover, none are food additives, so that safety and efficacy are not recognized.

### (Use)

The coronavirus-killing agent of the present disclosure has various applications including pharmaceutical and non-pharmaceutical applications.

Examples of pharmaceutical applications include killing of coronaviruses on the body surface tissue such as the skin or mucous membrane. The coronavirus-killing agent of the present disclosure has low irritability to the skin or mucous membrane. The killing agent can be used in medical settings, etc. for sterilizing hands, fingers, etc. of physicians, nurses, dentists, pharmacists, veterinarians, etc. The killing agent can be used to kill coronaviruses on a human body, livestock, pet, etc.

Examples of non-pharmaceutical applications include sterilization of the surface of everyday products, etc. The killing agent can be used on cooking utensils (including those made of wood), containers, chopping boards, strainers, cooking machines, sinks, fingers/hands of an operator, kitchens, doorknobs, work benches, knives, dishes (including chopsticks), sponges, rags, sinks, bathrooms, drains, drain pipes, toilets, toilet seats, toilet paper holders, hand washing facilities, living space (including restroom) walls, floors, ceilings, tables, furniture, toys, baby bottles, breast pumps, milled rice, legumes, fruits, seaweed, fresh fish or shellfish (including whale meat), meat, meat products, whale meat products, food products including those preserved by salting, drying, or other methods, etc.

### (Killing agent)

The present disclosure discloses use of a chlorous acid aqueous solution as a coronavirus-killing agent. The present disclosure can be used for killing coronaviruses at any location where coronaviruses can be present. The coronavirus killing agent of the present disclosure can sustain stable sterilizing power, so that the killing agent can be used in various applications.

### (Killing method)

The present disclosure discloses a method of killing coronaviruses with a chlorous acid aqueous solution. A coronavirus-killing agent comprising a chlorous acid aqueous solution of the present disclosure can be used directly as an undiluted solution or after suitable dilution in accordance with the application.

A kitchen, sink, doorknob, workbench, etc. can be washed with a neutral detergent, etc., then rinsed off with flowing water, sprayed with the coronavirus-killing agent of the present disclosure without dilution as an undiluted solution, then wiped with a rag, etc.

A cooking utensil, cutting board, knife, dish, etc. can be washed with a dish detergent, etc., then rinsed off with flowing water, and immersed in a solution of the coronavirus killing agent of the present disclosure diluted 2- to 10-fold or 2- to 20-fold for 10 minutes or longer, or 30 minutes if possible. Alternatively, an undiluted solution of the coronavirus killing agent of the present disclosure can be directly sprayed (rule of thumb: twice per 10 cm²), left standing for about 5 minutes, 10 minutes or longer, or 30 minutes, then rinsed off with flowing water, and dried.

Sponges, rags, etc. can be washed with a neutral detergent then rinsed off with flowing water, and immersed for 30 minutes in a solution of the coronavirus-killing agent of the present disclosure diluted 2- to 10-fold. Moisture can then be removed and directly dried.

For a cooking machine, parts are removed from the main body of the machine, washed with a neutral detergent, and rinsed off with flowing water, and the part can be immersed in a solution of the coronavirus killing agent of the present disclosure diluted 2- to 20-fold for 10 minutes or longer, or 30 minutes if possible. Alternatively, an undiluted solution can be sprayed, left standing for 10 minutes or longer, or 30 minutes if possible, and wiped off. The main body of the machine can be sprayed with an undiluted solution of the coronavirus-killing agent of the present disclosure, left standing for 10 minutes or longer, or 30 minutes if possible, and wiped off. Alternatively, the main body can be wiped off with a rag, etc., containing a solution of the coronavirus killing agent of the present disclosure diluted 2- to 20-fold.

Workbenches can be washed with a neutral detergent, etc., rinsed off with flowing water, then sprayed with an undiluted solution of the coronavirus killing agent of the present disclosure, left standing for 10 minutes or longer, or 30 minutes if possible, and wiped off. Alternatively, workbenches can be wiped off with a rag, etc., containing a solution of the coronavirus killing agent of the present disclosure diluted 2- to 20-fold.

Dishes (under normal circumstances) can be washed with a dishwashing detergent, immersed in a solution of the coronavirus-killing agent of the present disclosure diluted 20-fold for 30 minutes or longer, washed with water, and dried and stored in a hot air disinfecting storage.

Dishes (when suspected of an infection or during a sustained period of infection) can be immersed in an undiluted solution of the coronavirus killing agent of the present disclosure for 30 minutes or longer, washed with a detergent, immersed in a solution of the coronavirus killing agent of the present disclosure diluted 10-fold for 30 minutes or longer, washed with water, and dried and stored in a hot air disinfecting storage.

Baby bottles and breast pumps can be disassembled, immersed in warm water, washed with a neutral detergent, rinsed off with flowing water, and immersed in a solution of the coronavirus-killing agent of the present disclosure diluted 2- to 20-fold for 60 minutes or longer, or 2 to 3 hours if possible.

Sinks and bathrooms can be washed with a neutral detergent, etc., rinsed off with flowing water, and sprayed with an undiluted solution of the coronavirus killing agent of the present disclosure without dilution, and subsequently rinsed off with flowing water, or thoroughly wiped off with a fabric, unwoven fabric, etc.

For drains, drain pipes, etc., an undiluted solution of the coronavirus killing agent of the present disclosure can be directly poured in, left standing for about 5 minutes, and then thoroughly rinsed off with flowing water.

Toilets, toilet seats, toilet paper holders, doorknobs, hand washing facilities, etc. can be washed with a bathroom detergent, etc. then sprayed directly (rule of thumb: 2 or 3 times) with an undiluted solution of the coronavirus killing agent of the present disclosure, and subsequently wiped with fabric, unwoven fabric, paper towel, etc. Alternatively, the coronavirus-killing agent of the present disclosure may be diluted 2- to 4-fold (but may be a solution diluted 2- to 10-fold for routine disinfection), and a clean fabric or unwoven fabric may be soaked with the solution for wiping.

Bathroom walls, floors, ceilings, etc. can be cleanly washed with a bathroom detergent, etc., then mopped while being sprayed directly (rule of thumb: 5 times per 1 m²) with an undiluted solution of the coronavirus-killing agent of the present disclosure. Alternatively, the coronavirus killing agent of the present disclosure may be diluted 2-to 4-fold (but may be a solution diluted 2- to 10-fold for routine disinfection), and a mop, rag, etc. may be thoroughly soaked with the solution for thorough wiping.

Vomit, etc. (wet substance) can be treated by wiping the vomit, etc. (wet substance) with a disposable fabric, etc., placing the waste in a plastic bag, and pouring the undiluted solution of the coronavirus-killing agent of the present disclosure thereon. The floor and the surrounding area to which the vomit, etc. (wet substance) is adhering can be covered with a fabric, etc. soaked in an undiluted solution of the coronavirus-killing agent of the present disclosure or wiped to immerse the floor and the surrounding area therewith.

Reference literature such as scientific literature, patents, and patent applications cited herein are incorporated herein by reference to the same extent that the entirety of each document is specifically described.

As described above, the present disclosure has been described while showing preferred embodiments to facilitate understanding. The present disclosure is described hereinafter based on Examples. The above descriptions and the following Examples are not provided to limit the present invention, but for the sole purpose of exemplification. Thus, the scope of the present invention is not limited to the embodiments and Examples specifically described herein and is limited only by the scope of claims.

### [Examples]

### (Quantification method of chlorous acid aqueous solution)

About 5 g of the present product is precisely measured. Water is added thereto so that the solution is exactly 500 ml to prepare a sample solution. After 20 ml of the sample solution is accurately measured, placed in an iodine flask, and combined with 10 ml of sulfuric acid (1→10), 1 g of potassium iodide is added thereto. The flask is immediately sealed and shaken well. 5 ml of a potassium iodide reagent is poured into the top portion of the iodine flask and left standing in the dark for 15 minutes. The plug is then loosened to pour in a potassium iodide reagent and the flask is sealed immediately. After thoroughly mixing, freed iodine is titrated with 0.1 mol/L sodium thiosulfate (indicator: 5 ml of starch reagent). The starch reagent is added when the color of the solution has changed to a light yellow color near the endpoint, where the end point is when blue color of the solution disappears. A blank test is separately conducted for correction (1 mL of 0.1 mol/L sodium thiosulfate solution = 1.711 mg of HClO₂).

### (Manufacturing Examples)

Chlorous acid aqueous solution formulations used in the following Examples were manufactured as follows. Chlorous acid aqueous solution may be abbreviated as "CAAS" herein, but the terms are synonymous.

### Component analysis table for chlorous acid aqueous solution

**[Table 3A]**

| CAAS specification | Specification Value | Match/Not a Match |
|---|---|---|
| Content | 4-6 % | 4.1% |
| Attribute | light yellowish green to yellowish red | yellowish red |
| Confirmation Test (1) | When 0.1 ml of potassium permanganate solution (1→300) is added to 5 ml of an aqueous solution of the present product (1→20), the solution turns reddish purple. When 1 ml of sulfuric acid (1→20) is added thereto, the solution turns light yellow. | Match |
| Confirmation Test (2) | An aqueous solution of the present product (1→20) has maximum absorbance sections at wavelengths 258 to 262 nm and 346 to 361 nm. | Match |
| Confirmation Test (3) | If potassium iodide starch paper is dipped in the present product, the potassium iodide starch paper changes to a blue color and then the color fades. | Match |
| Purity Test (1) | 1.0 µg/g or lower for lead | Match |
| Purity Test (2) | 1.0 µg/g or lower for Ag₂O₃ | Match |

A chlorous acid aqueous solution formulation was manufactured using this chlorous acid aqueous solution based on the following composition.

**[Table 3B]**

| | Raw material name | Blended amount | Concentration | Acceptable range |
|---|---|---|---|---|
| 1 | Tap water | 258.0 g | | |
| 2 | Dipotassium hydrogen phosphate | 17.0 g | 1.70% | 0.70% to 13.90% |
| 3 | Potassium hydroxide | 5.0 g | 0.50% | 0.10% to 5.60% |
| 4 | Chlorous acid aqueous solution (pH 2.9 to 3.5) | 720.0 g | 72.00% | 0.25% to 75% |
| Total | | Chlorous acid 30000 ppm | 1000 g | |

**[Table 3C]**

| CAAS Specification | Chlorous acid aqueous solution formulation manufactured with chlorous acid aqueous solution |
|---|---|
| Content | 3.0% |
| Attribute | Yellow |
| Confirmation Test (1) | Match |
| Confirmation Test (2) | Match |
| Confirmation Test (3) | Match |
| Purity Test (1) | Match |
| Purity Test (2) | Match |

For the "chlorous acid aqueous solution formulation manufactured with chlorous acid aqueous solution" prepared based on the preparation method described above, the concentration of "chlorous acid aqueous solution" was measured based on the "Quantification method of chlorous acid aqueous solution" described above, and buffer prepared to achieve the free chlorine concentration described in each Example (phosphate buffer comprising dipotassium hydrogen phosphate or potassium dihydrogen phosphate) was used to prepare the chlorous acid aqueous solution of each Example.

### (Chlorous acid aqueous solution efficacy confirmation test)

A test was conducted on an antiviral effect of a chlorous acid aqueous solution against SARS-CoV-2 (2019 novel coronavirus).
Tested subject: Chlorous Acid N Barrier (HonbuSankei): [Components/quantity] 20g of chlorous acid aqueous solution in 100 mL; content of 0.8% as (chlorous acid HClO₂ = 68.46) [as of manufacture], free chlorine concentration of 200 mg/L or greater (as Cl = 35.45)
Virus: SARS-CoV-2 (2019-nCoV/Japan/AI/I-004/2020) strain (provided by the National Institute of Infectious Diseases)
Cells: VeroE6/TMPRSS2 cells (JCRB1819)
FBS concentration in viral solution: 0%
Reagents: Dulbecco's Modified Eagle Medium (DMEM) (Fujifilm Wako Pure Chemical), Hipolypepton N (Fujifilm Wako Pure Chemical), bovine serum albumin, Fraction V, special grade (Katayama Chemical Industries)
Testing method: Chlorous Acid N Barrier was diluted with distilled water by using a polystyrene tube, and reacted with a virus within the polystyrene tube.

A 10% (w/v) aqueous solution of protein-loading polypeptone was prepared and filtered/sterilized through a 0.1 um filter. The same amount of viral solution was mixed therewith and used for a test. The polypeptone concentration in the mixture with viruses was 5%, and the viruses were diluted to 1/2. When the viral mixture and a reagent such as Chlorous Acid N Barrier were mixed and reacted at a ratio of (1:9), the final concentration of polypeptone in a reaction solution was 0.5%. A 0.6% (w/v) aqueous solution of bovine serum albumin (BSA) was similarly prepared so that the final concentration in the reaction solution would be 0.03%.

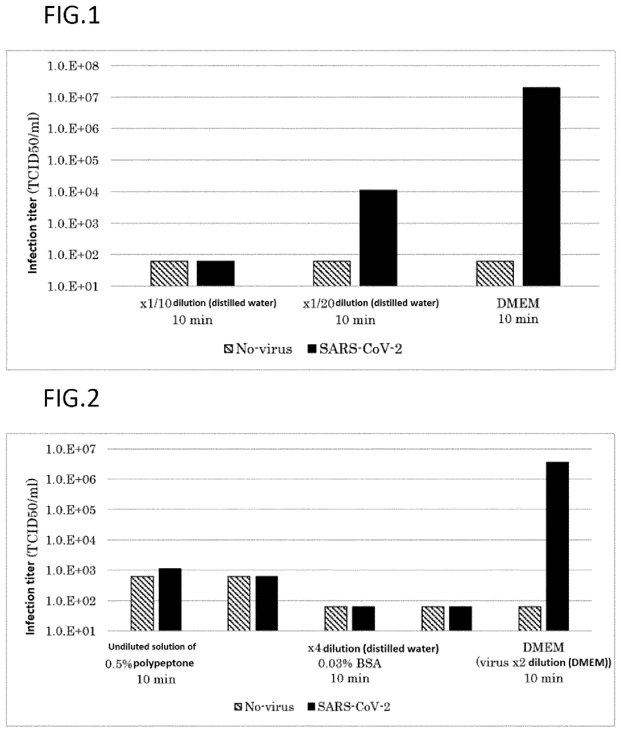

The viruses and reagent were mixed at a ratio of (1:9) and reacted for a predetermined time at room temperature, then diluted 10-fold with DMEM to stop the reaction, then the mixture was serially diluted 10-fold to measure the viral infection titer by TCID₅₀ (Table 4, Figure 1).

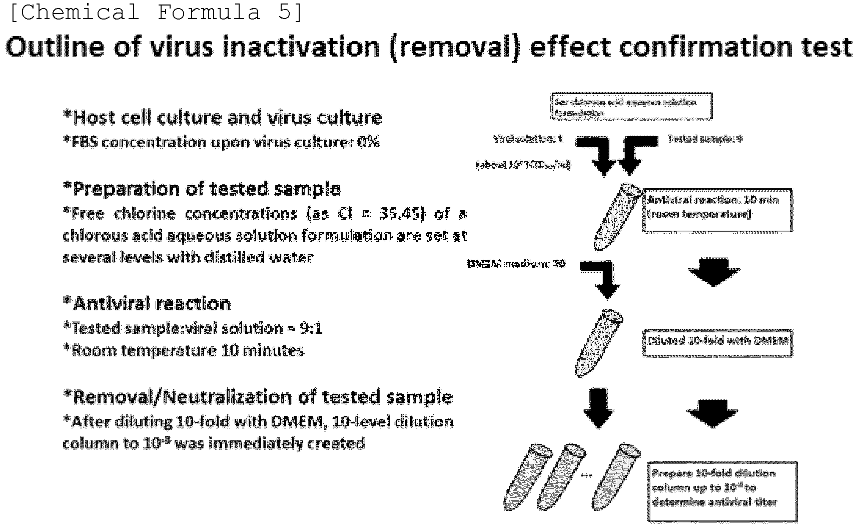

### Results:

### [Antiviral test]

**[Table 4]**

| Test reagent/reaction condition | No-virus | SARS-CoV-2 | log | Δlog |
|---|---|---|---|---|
| x1/10 dilution (distilled water), 10 min | 6.3.E+01 | 6.3.E+01 | 1.8 | -5.50 |
| x1/20 dilution (distilled water), 10 min | 6.3.E+01 | 1.1. E+04 | 4.1 | -3.25 |
| DMEM, 10 min | 6.3.E+01 | 2.0.E+07 | 7.3 | standard |

Virus infection titer decreased to the detection limit with 1/10 diluent of Chlorous Acid N Barrier. Since the infection titer was at the detection limit, and virus-infected cells could not be observed at all, the actual infection titer is understood to be lower, so that viruses can be understood to be completely inactivated. 1/20 diluent of Chlorous Acid N Barrier decreased the viral infection titer to 1/1000 or less compared to the untreated sample (DMEM), and is thus understood to have inactivation capacity.

### [Protein loaded antivirus test]

**[Table 5]**

| Test reagent/reaction condition | No-virus | SARS-CoV-2 | log | Δlog |
|---|---|---|---|---|
| Undiluted solution, 0.5% polypeptone, 10 min | 6.3.E+02 | 1.1. E+03 | 3.1 | -3.5 |
| x2 dilution (distilled water), 0.03% BSA, 10 min | 6.3.E+02 | 6.3.E+02 | 2.8 | -3.8 |
| x4 dilution (distilled water), 0.03% BSA, 10 min | 6.3.E+01 | 6.3.E+01 | 1.8 | -4.8 |
| x8 dilution (distilled water), 0.03% BSA, 10 min | 6.3.E+01 | 6.3.E+01 | 1.8 | -4.8 |
| DMEM, (virus x2 dilution (DMEM)), 10 min | 6.3.E+01 | 3.6.E+06 | 6.6 | standard |

With an undiluted solution of Chlorous Acid N Barrier, only one well had viral infection in the presence of 0.5% polypeptone. While the effect thereof did not reach the detection limit, viruses were strongly inactivated, and viral infection titer decreased to 1/1000 or less. However, in view of the near complete viral inactivation with the aforementioned 1/10 diluent of Chlorous Acid N Barrier, it is inferred that the effect of Chlorous Acid N Barrier is attenuated by protein loading (Table 5, Figure 2).

In the presence of 0.03% BSA, viruses were inactivated to the detection limit by each of x2, x4, or x8 diluents of Chlorous Acid N Barrier, and an effect of protein loading was not observed.

Chlorous Acid N Barrier completely inactivated SARS-CoV-2 under the conditions of 1/10 dilution and 10 minute reaction.

As described above, the present invention is exemplified by the use of its preferred embodiments. It is understood that the scope of the present invention should be interpreted solely based on the claims. The present application claims priority to Japanese Patent Application No. 2020-125824 (filed on July 22, 2020). It is understood that the entire content thereof is incorporated herein by reference. It is understood that any patent, any patent application, and any references cited herein should be incorporated herein by reference in the same manner as the contents are specifically described herein.

### [Industrial Applicability]

The present disclosure provides coronavirus killing.

## Claims

1. An agent for use in killing coronavirus comprising a chlorous acid aqueous water.

2. The agent for use in killing of claim 1, wherein a free chlorine concentration (as Cl = 35.45) of the chlorous acid aqueous solution is at least 1 mg/L in the absence of an organic matter.

3. The agent for use in killing of claim 1, wherein chlorous acid content (as HClO₂ = 68.46) of the chlorous acid aqueous solution is at least 100 ppm in the absence of an organic matter.

4. The agent for use in killing of claim 1, wherein a free chlorine concentration (as Cl = 35.45) of the chlorous acid aqueous solution is at least 5 mg/L in the presence of an organic matter including disinfection of a hand, finger, etc.

5. The agent for use in killing of claim 1, wherein chlorous acid content (as HClO₂ = 68.46) of the chlorous acid aqueous solution is at least 200 ppm in the presence of an organic matter including disinfection of a hand, finger, etc.

6. The agent for use in killing of claim 1, wherein a free chlorine concentration (as Cl = 35.45) of the chlorous acid aqueous solution is at least 10 mg/L in the presence of an organic matter including a contaminant, etc. equivalent to 0.5% BSA or greater.

7. The agent for use in killing of claim 1, wherein chlorous acid content (as HClO₂ = 68.46) of the chlorous acid aqueous solution is at least 400 ppm in the presence of an organic matter including a contaminant, etc. equivalent to 0.5% BSA or greater.

8. The agent for use in killing of claim 7, wherein a specification value of the chlorous acid aqueous solution is 4 to 6%.

9. The agent for use in killing of any one of claims 1 to 8, wherein the coronavirus is a Letovirinae or Orthocoronavirinae virus.

10. The agent for use in killing of any one of claims 1 to 9, wherein the coronavirus is a virus of the genus alphacoronavirus, betacoronavirus, gammacoronavirus, or deltacoronavirus.

11. The agent for use in killing of any one of claims 1 to 10, wherein the coronavirus is a virus of the genus betacoronavirus.

12. The agent for use in killing of any one of claims 1 to 11, wherein the coronavirus is a virus of the subgenus Colacovirus, Decacovirus, Duvinacovirus, Luchacovirus, Minacovirus, Minunacovirus, Myotacovirus, Nyctacovirus, Pedacovirus, Rhinacovirus, Setracovirus, Soracovirus, Sunacovirus, Tegacovirus, Embecovirus, Hibecovirus, Merbecovirus, Nobecovirus, Sarbecovirus, Andecovirus, Buldecovirus, Herdecovirus, Brangacovirus, Cegacovirus, or Igacovirus.

13. The agent for use in killing of any one of claims 1 to 12, wherein the coronavirus is a coronavirus that infects a human.

14. The agent for use in killing of any one of claims 1 to 13, wherein the coronavirus is HCoV-HKU1, HCoV-OC43, SARS coronavirus (SARS-CoV), MERS coronavirus (MERS-CoV), or 2019 novel coronavirus (SARS-CoV-2).

15. The agent for use in killing of any one of claims 1 to 14, wherein the coronavirus is SARS coronavirus (SARS-CoV), MERS coronavirus (MERS-CoV), or 2019 novel coronavirus (SARS-CoV-2) .

16. The agent for use in killing of any one of claims 1 to 15, wherein the agent for use in killing is an agent for use in killing for hands and fingers, and a free chlorine concentration (as Cl = 35.45) of the chlorous acid aqueous solution is at least 5 mg/L.

17. A method of killing a coronavirus by using a chlorous acid aqueous solution.

18. The method of claim 17, wherein the coronavirus is contacted with the chlorous acid aqueous solution in the absence of an organic matter.

19. The method of claim 17 or 18, wherein a free chlorine concentration (as Cl = 35.45) of the chlorous acid aqueous solution is at least 1 mg/L.

20. The method of any one of claims 17 to 19, wherein the coronavirus is contacted with the chlorous acid aqueous solution in the presence of an organic matter.

21. The method of any one of claims 17 to 20, wherein a free chlorine concentration (as Cl = 35.45) of the chlorous acid aqueous solution is at least 10 mg/L.

22. A chlorous acid aqueous solution for use in killing a coronavirus.

23. Use of a chlorous acid aqueous solution as an agent for use in killing coronavirus.

24. Use of a chlorous acid aqueous solution for the manufacture of an agent for use in killing coronavirus.
